## Europäisches Patentamt

## European Patent Office

## Office européen des brevets

(19)

(11) Veröffentlichungsnummer: **0 137 428**
**B1**

(12) # EUROPÄISCHE PATENTSCHRIFT

(45) Veröffentlichungstag der Patentschrift:
10.05.89

(51) Int. Cl.⁴: **A 61 L 2/04**, A 61 K 35/16

(21) Anmeldenummer: **84111601.5**

(22) Anmeldetag: **28.09.84**

(54) **Verfahren zur Pasteurisierung von Plasma oder von Konzentraten der Blutgerinnungsfaktoren II, VII, IX, und X.**

(30) Priorität: **06.10.83 DE 3336631**

(43) Veröffentlichungstag der Anmeldung:
**17.04.85 Patentblatt 85/16**

(45) Bekanntmachung des Hinweises auf die Patenterteilung:
**10.05.89 Patentblatt 89/19**

(84) Benannte Vertragsstaaten:
**AT BE CH DE FR GB IT LI LU NL SE**

(56) Entgegenhaltungen:
**EP-A-0 050 061**
**EP-A-0 052 827**
**EP-A-0 053 338**
**EP-A-0 103 196**
**EP-A-0 106 269**

**CHEMICAL ABSTRACTS, Band 66, Nr. 3, 16. Januar 1967, Seite 881, Nr. 9221p, Columbus, Ohio, US; M. STEINBUCH u.a.: "Hemostatic fraction of the plasma containing four factors (II, VII, IX, X) of the blood coagulation system" & PROBL. GEMATOL. PERELIV. KROVI 11(10), 15-21 (1966)**

**Die Akte enthält technische Angaben, die nach dem Eingang der Anmeldung eingereicht wurden und die nicht in dieser Patentschrift enthalten sind.**

(73) Patentinhaber: **BEHRINGWERKE Aktiengesellschaft, Postfach 1140, D-3550 Marburg 1 (DE)**

(72) Erfinder: **Heimburger, Norbert, Prof. Dr., Sonnenhang 10, D-3550 Marburg (DE)**
Erfinder: **Kumpe, Gerhardt, Aspherfeld 14, D-3552 Wetter (DE)**
Erfinder: **Wormsbächer, Wilfried, Blumenweg 9, D-3570 Kirchhain (DE)**
Erfinder: **Preis, Hans Martin, Am Wäldchen 4, D-3550 Marburg (DE)**

(74) Vertreter: **Meyer-Dulheuer, Karl-Hermann, Dr., HOECHST Aktiengesellschaft Zentrale Patentabteilung Postfach 80 03 20, D-6230 Frankfurt/Main 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung einer von aktiven Viren praktisch freien Plasma-Präparation oder eines von solchen Viren praktisch freien Konzentrates der Blutgerinnungsfaktoren II, VII, IX und X durch Erwärmen in Gegenwart von Stabilisatoren sowie eine danach hergestellte Präparation dieser Faktoren. Solche Präparationen können zur Behandlung von Blutgerinnungsstörungen verwendet werden.

Die Blutgerinnung ist ein komplexer, in Stufen ablaufender Vorgang, der durch verschiedene physiologische wie pathologische Ursachen ausgelöst wird, und dessen Ablauf von etwa 20 fördernden oder hemmenden Faktoren abhängt. Durch Verminderung oder Vermehrung dieser Blutgerinnungsfaktoren treten Störungen der Blutgerinnung auf, die sich teilweise als Krankheiten manifestieren.

Konzentrate der Faktoren II, VII, IX und X sind für die Behandlung verschiedener angeborener oder erworbener Störungen der Synthese dieser Faktoren geeignet. Die Behandlung von Patienten mit Konzentraten dieser Faktoren war bisher mit dem Risiko einer Virusübertragung und speziell einer Hepatitis verbunden.

Albumin gilt als hepatitissicher, wenn es in wassriger Lösung und in Gegenwart von Stabilisatoren auf 60° C erhitzt wird (Gellis, S. S. et al., J. Clin. Invest. 27, (1948) 239). Es ist deshalb anzunehmen, daß ein in Gegenwart geeigneter Stabilisatoren erhitztes Konzentrat der Faktoren II, VII, IX und X ebenfalls hepatitissicher ist.

In der deutschen Offenlegungsschrift 2 916 711 ist ein Verfahren zur Stabilisierung anderer Gerinnungsfaktoren in wässriger Lösung gegen Wärme durch Zusatz einer Aminosäure und eines Mono- oder Oligosaccharids oder Zuckeralkohols beschrieben.

Doch kann auf diese Weise nicht verhindert werden, daß die Faktoren II, VII, IX und X beim Erwärmen zum großen Teil inaktiviert werden. Daher war die Beobachtung ein Fortschritt, daß die Faktoren II und VII durch Chelat-Bildner (DE-3 043 857 AI) und die Faktoren IX und X durch Calcium (DE-3 045 153 AI) vor einer thermischen Inaktivierung in wässriger Lösung geschützt werden. Beide Verfahren erlaubten jedoch nicht die Herstellung einer Präparation aller vier Faktoren in einem Arbeitsprozeß, wie sie wünschenswert ist, da alle vier Faktoren bei Vitamin K-Mangel oder unter der Therapie mit oralen Antikoagulantien vermindert sind und daher gleichzeitig substituiert werden müssen.

Es bestand demnach die Aufgabe, ein Verfahren zur Stabilisierung einer die Faktoren II, VII, IX und X enthaltenden wässrigen Lösung gegen thermische Inaktivierung zu finden.

Überraschend wurde nun gefunden, daß eine alle vier Faktoren II, VII, IX und X enthaltende wässrige Lösung durch Zusatz von Calciumionen und eines Chelat-Bildners gegen die nachteiligen Folgen einer Wärmebehandlung geschützt werden kann. Gegebenenfalls können Antithrombin III (AT) und Heparin zugesetzt werden, um eine Aktivierung der Faktoren II und VII zu verhindern.

Damit waren die Voraussetzungen geschaffen, diese vier Faktoren frei von aktiven Viren in einem Arbeitsgang mit hoher Reinheit und Ausbeute herzustellen.

Der Chelat-Bildner wird zusätzlich zu dem bereits vorhandenen Citrat zugesetzt.

Es überrascht, daß eine Mischung von Calciumionen und Chelat-Bildner den angestrebten Schutz aller Faktoren II, VII, IX und X gegen einen Aktivitätsverlust durch Erwärmen bewirkt, obwohl nach DE-3 043 857 ein Chelat-Bildner für den Schutz der Faktoren II und VII zweckmäßig ist, während zum gleichen Zweck für die Faktoren IX und X Calciumionen gebraucht werden (DE-3 045 153), und Chelat-Bildner auch mit Calciumionen Komplexe bilden. Je nachdem, ob der Chelat-Bildner oder die Calciumionen im Überschuß vorliegen, sollten deshalb jeweils nur die beiden entsprechenden Faktoren gegen eine Wärmeabbau geschützt sein und nicht alle vier.

Gegenstand der Erfindung ist daher ein Verfahren zur Herstellung einer praktisch virusfreien und hepatitissicheren Präparation der Blutgerinnungsfaktoren II, VII, IX und X durch Erwärmen einer wässrigen Lösung, gegebenenfalls in Gegenwart einer Aminosäure und/oder eines Saccharids oder Zuckeralkohols, dadurch gekennzeichnet, daß in Gegenwart von Calciumionen und einem Chelat-Bildner und gegebenenfalls von Antithrombin III und/oder Heparin erwärmt wird.

Eine solche Lösung kann eine diese Faktoren enthaltende Lösung oder ein diese enthaltendes Konzentrat, aber auch Plasma oder eine Plasma- oder Placentafraktion sein.

Die optimale Konzentration von Calciumionen liegt im Bereich von 1 - 50 mmol/l, vorzugsweise 25 - 50 mmol/l.

Geeignete Calciumionen liefernde Salze sind beispielsweise das Chlorid, Acetat oder Nitrat sowie alle wasserlöslichen Calcium-Salze von Zuckersäuren wie Gluconsäure oder Lactonsäure. Bevorzugt werden das Chlorid und das Acetat verwendet.

Geeignete Chelat-Bildner sind beispielsweise: Ethylendiamino-tetraessigsäure (EDTA), Ethylenglycol-bis-(2-aminoethylether)-tetraessigsäure (EGTA), Diaminocyclohexan-tetraessigsäure (CDTA), Diaminopropantetraessigsäure oder Nitrilotriessigsäure und besonders deren lösliche Alkali-Salze.

Bevorzugt werden aliphatische Aza-tri- oder tetra-Carbonsäuren mit 6 - 20 Kohlenstoffatomen und 1 oder 2 Stickstoffatomen und deren lösliche Alkalisalze und besonders die Natriumsalze von Ethylendiamino-tetraessigsäure (EDTA) oder Ethylenglycol-bis-(2-aminoethylether)-tetraessigsäure (EGTA) verwendet. Die optimale Konzentration der Chelat-Bildner ist 1 bis 20 mmol/l und vorzugsweise 5 mmol/l.

Als besonders geeignet haben sich Mischungen von 25 mmol/l Calcium mit 5 mmol/l EDTA erwiesen.

Antithrombin III wird in einer Konzentration von 0,05 - 2 E/ml, bezogen auf die Aktivität von 1 ml Citratmischplasma (1 E), eingesetzt und zusammen mit Heparin in Konzentrationen von 0,5 - 20 USP-E/ml, bevorzugt 0,2 E Antithrombin III mit 2 USP-E Heparin.

2

In Anwesenheit einer Mischung von Calciumionen und einem Chelat-Bildner und gegebenenfalls des Antithrombin III-Heparin-Komplexes kann die wässrige Lösung der Gerinnungsfaktoren so lange erhitzt werden, daß eine Übertragung von Viren und speziell Hepatitis-Erregern nach dem Stand des heutigen Wissens praktisch auszuschließen ist. Dies gilt vor allem, wenn die Pasteurisierung mit Adsorptions- und Fällungsverfahren kombinert wird, bei denen die Wirkstoffe im Überstand bleiben und die Hepatitisviren zusammen mit dem unlöslichen Niederschlag abgetrennt werden können. Eine Präparation, die mindestens 10 Stunden bei etwa 60°C in wässriger Lösung gehalten wurde, gilt heute als praktisch hepatitissicher insbesondere wenn von humaner Gewebeflüssigkeit ausgegangen wird, in der nach einem Test der dritten Generation Hepatitisviren nicht nachgewiesen werden können.

Eine besonders bevorzugte Ausführungsform der Erfindung ist dadurch gekennzeichnet, daß eine alle vier Faktoren enthaltende Lösung, vorzugsweise Plasma oder eine Plasma- oder Plazentafraktion, mit 0,2 - 2 E/ml Antithrombin III, 2 - 20 USP-E/ml Heparin, 25 - 50 mmol/l Calciumionen und 1 - 20 mmol/l EDTA, 1 - 3 mol/l mindestens einer der Aminosäuren Glycin, alpha- oder beta-Alanin, Lysin, Leucin, Valin, Asparagin, Serin, Hydroxyprolin, Prolin, Glutamin, alpha-, beta- oder gamma-Aminobuttersäure, vorzugsweise aber Glycin und 20 bis 60 g/100 g Lösung eines Mono- oder Oligosaccharids oder Zuckeralkohols, vorzugsweise 1 bis 3 mol/l Glycin und 20 bis 60 g/100 g Lösung Saccharose, versetzt, auf eine Temperatur von 30°C bis 100°C, vorzugsweise 60°C bis 100°C, erhitzt und 1 Minute bis 48 Stunden, vorzugsweise 8 - 12 Stunden, bei dieser Temperatur gehalten wird, wobei die kürzeste Zeit der höchsten Temperatur zuzuordnen ist und umgekehrt.

Es wird ein pH-Wert von 6 bis 8 eingehalten. Auf diese Weise wird eine praktisch virusfreie Präparation der Faktoren II, VII, IX und X erhalten.

Abhängig von der Löslichkeit des Calciumsalzes, der Aminosäure oder des Kohlenhydrates können die entsprechenden Konzentrationen von 0,3 und 3,0 mol/l und 60 g/100 g zu höheren Konzentrationen erweitert werden, wenn das Calciumsalz, die Aminosäure oder das Kohlenhydrat bei der gewünschten Temperatur eine entsprechend höhere Löslichkeit aufweisen. Die Temperaturbehandlung kann auch in mehreren aufeinanderfolgenden Schritten durchgeführt werden.

Mit der bevorzugt verwendeten Kombination von Antithrombin III, Heparin, Calciumchlorid und EDTA mit Glycin und Saccharose wird durch Erhitzen ein hepatitissicheres Präparat erzielt.

Um zu einem Virus-freien Plasma zu kommen, das die Faktoren II, VII, IX und X in nativer und aktiver Form enthält, wird das Plasma mit der Mischung eines Chelatbildners, vorzugsweise EDTA und Calciumionen, vorzugsweise in solchen Mengen, daß diese in den Konzentrationen von 5 mmol/l EDTA und 25 mmol/l Calciumchlorid vorliegen, versetzt und in einer Saccharose (60 g/100 g)-Glycin (2 mol/l)-Mischung auf 60°C erwärmt und 10 Stunden bei dieser Temperatur gehalten.

Um zu einem nativen und aktiven Konzentrat der Faktoren II, VII, IX und X zu kommen, wird die Plasma- oder Placentafraktion, zweckmäßigerweise eine Fraktion, in der die zu stabilisierenden Faktoren angereichert sind, mit einer Mischung eines Chelatbildners, vorzugsweise EDTA und Calciumionen, vorzugsweise in den Konzentrationsverhältnissen von 5 mmol/l EDTA und 25 mmol/l Calciumchlorid, versetzt. Nach Zugabe von Antithrombin III, vorzugsweise 0,2 E/ml und Heparin, vorzugsweise 2 USPE/ml, wird in einei Saccharose (60 g/100 g)-Glycin (2 mol/l)-Mischung auf 60°C erwärmt und 10 h bei dieser Temperatur gehalten.

Eine solche Fraktion erhält man beispielsweise nach dem Verfahren von Soulier et al., Thrombosis Diath. Haemorrh. Suppl. 35, (1969) 61. Dazu adsorbiert man Plasma, das aus einem mit 0,01 mol/l EDTA anticoaguliertem Blut gewonnen wurde, an Calciumphosphat und zentrifugiert ab. Dabei werden die Faktoren quantitativ an das Adsorbens gebunden und können durch mehrfaches Eluieren mit 0,2 mol/l Tri-Natriumcitrat gewonnen werden. Die vereinigten Eluate werden durch kombinierte Alkohol- und Essigsäurefällungen bei Temperaturen von -8°C bis +4°C weiter gereinigt. Dabei werden die Faktoren gleichzeitig konzentriert.

Man nimmt das Konzentrat in einem geeigneten Puffer, vorteilhaft Kochsalz/Natriumcitrat mit 0,06 bzw. 0,02 mol/l und pH 7,5 auf.

Die Rückgewinnung und Reinigung der Gerinnungsfaktoren aus der erhitzten Lösung kann durch Fällen mit Ammoniumsulfat bei 30 - 45 % Sättigung, Absorbieren des Überstandes an 0,4 bis 1 g/100 ml Calciumphosphat und Eluieren erreicht werden.

Die Kontrolle der Maßnahmen zur Anreicherung und Reinigung der Faktoren II, VII, IX und X sind dem Fachmann durch die Kenntnis von Bestimmungsmethoden für die betreffenden Substanzen geläufig. Unter Verwendung dieser Kontrollmethoden können die Verfahrensbedingungen unter dem Gesichtspunkt einer befriedigenden Ausbeute und einer befriedigenden Reinheit des Produktes gelenkt werden.

Der Faktor II kann zum Beispiel nach der Methode von Koller, F. et al., Dtsch. med. Wschr. 81, (1956) 516, bestimmt werden. Dazu werden ein Teil, beispielsweise 0,1 ml Faktor II-Mangelplasma, und ein Teil verdünntes Normalplasma vermischt. Diese Mischung wird 30 Sekunden bei +37°C gehalten. Danach setzt man zwei Teile calciumhaltiges Thromboplastin, beispielsweise hergestellt nach der deutschen Patentschrift 2 356 493, zu und bestimmt die Zeit, die bis zum Auftreten eines Gerinnsels verstreicht. Zur quantitativen Aussage wird die aus der Faktor II enthaltenden Lösung sich ergebende Gerinnungszeit unter Bezugnahme auf eine mit einer Normalplasma-Verdünnungsreihe erzielten Eichkurve abgelesen.

Eine Einheit Faktor II entspricht der Faktor II-Aktivität von 1 ml Normalplasma.

Der Faktor VII kann zum Beispiel nach der Methode von Koller, F. et al., Acta haemat. 6, (1951) 1, bestimmt werden. Dazu werden ein Teil, beispielsweise 0,1 ml Faktor VII-Mangelplasma und ein Teil verdünntes Normalplasma vermischt. Diese Mischung wird 30 Sekunden bei +37°C gehalten. Danach setzt man zwei Teile calciumhaltiges Thromboplastin, beispielsweise hergestellt nach der deutschen Patentschrift 2 356 493, zu und bestimmt die Zeit, die bis zum Auftreten eines Gerinnsels verstreicht. Zur quantitativen Aussage wird die aus der Faktor VII enthaltenden Lösung sich ergebende Gerinnungszeit unter Bezugnahme auf eine mit einer Normalplasma-Verdünnungsreihe erzielten Eichkurve abgelesen.

Eine Einheit Faktor VII entspricht der Faktor VII-Aktivität von 1 ml Normalplasma.

Die Bestimmung des Faktors IX erfolgt beispielsweise nach folgendem Verfahren:

1 Teil, beispielsweise 0,1 ml partielles Thromboplastin, zum Beispiel hergestellt nach der DE-AS-2 316 430, wird mit einem Teil Faktor IX-Mangelplasma und einem Teil verdünntem Normalplasma vermischt. Diese Mischung wird 6 Minuten bei 37°C gehalten. Nach Zusatz von einem Teil einer auf 37°C vorgewärmten 0,025 molaren Calciumchlorid-Lösung wird die Zeit bestimmt, die vom Zusatz der Calciumchlorid-Lösung bis zum Auftreten eines Gerinnsels verstreicht. Zur quantitativen Aussage wird die aus der Faktor IX enthaltenden Lösung sich ergebende Gerinnungszeit unter Bezugnahme auf eine mit einer Normalplasma-Verdünnungsreihe erzielten Eichkurve abgelesen.

1 Internationale Einheit (= 1 IE) Faktor IX entspricht der Faktor IX-Aktivität von 1 ml Normalplasma.

Der Faktor X kann beispielsweise nach der Methode von Duckert et al., Blood Coagulation, Hemorrhage and Thrombosis, Ed. Tocantins, L. M. and Kazal, L. A. (1964), bestimmt werden. Dazu werden ein Teil, zum Beispiel 0,1 ml Faktor X-Mangelplasma und 1 Teil verdünntes Normalplasma vermischt. Diese Mischung wird 30 Sekunden bei +37°C gehalten. Danach setzt man zwei Teile calciumhaltiges Thromboplastin, beispielsweise hergestellt nach der DE-PS-2 356 493, zu und bestimmt die Zeit, die bis zum Auftreten eines Gerinnsels verstreicht. Zur quantitativen Aussage wird die aus der Faktor X enthaltenden Lösung sich ergebende Gerinnungszeit unter Bezugnahme auf eine mit einer Normalplasma-Verdünnungsreihe erzielten Eichkurve abgelesen. Eine Einheit Faktor X entspricht der Faktor X-Aktivität von 1 ml Normalplasma.

Zur Abtötung der Hepatitisviren werden der Lösung Calciumionen, EDTA, Glycin und Saccharose und gegebenenfalls Antithrombin III und Heparin zugesetzt und erhitzt.

Nach Pasteurisierung (10 Stunden, 60°C) von Humanplasma nach Zusatz von Saccharose (60 g/100 g) und Glycin (2 mol/l) und in Gegenwart von 25 mmol/l Calciumchlorid sowie 5 mmol/l EDTA wurden folgende Aktivitäten, bezogen auf das eingesetzte Plasma, gefunden: F II 90 %, F VII 85 %, F IX 83 %, F X 80 %.

Der Einfluß der verschiedenen Stabilisatoren auf die Erhaltung der Aktivität der einzelnen Prothrombinfaktoren in einer Plasma- oder Placentafraktion beim Erhitzen ist der Tabelle I zu entnehmen. Die Pasteurisierung mit Saccharose und Glycin allein ergibt eine schlechte F IX-Ausbeute. Das ist jedoch sehr nachteilig, da das Prothrombin-Konzentrat vorwiegend für die Therapie der Hämophilie B eingesetzt wird. Die zusätzliche Verwendung von Calciumionen ohne Chelatbildner in der Konzentration von 6,25 mmol/l führt zu einer Aktivierung von F VII und F IX und vermindert auch die Ausbeuten an F II und F X. Eine solche Präparation wäre wegen eines möglichen Thromboserisikos für die Anwendung am Menschen nicht geeignet. Bei der Verwendung von EDTA ohne Calciumionen in der Konzentration von 5 mmol/l neben Saccharose und Glycin werden bei guten F II und F VII Ausbeuten schlechte F IX und F X Ausbeuten erzielt. Allein mit Antithrombin III, Heparin, Saccharose und Glycin lassen sich die Prothrombinfaktoren nicht stabilisieren.

Das beste Ergebnis ergibt die Kombination von Calciumionen mit EDTA bei Zusatz von Antithrombin III und Heparin mit Saccharose und Glycin. Unter diesen Bedingungen liegen die Ausbeuten für alle vier Faktoren in der Größenordnung von etwa 80 % und die Präparation ist auch frei von aktivierten Faktoren und Thrombin.

Die Konzentrationen an Gerinnungsfaktoren in einer wässrigen Lösung, mit der das beschriebene Verfahren ausgeführt werden kann, liegen besonders in den folgenden Bereichen:

| | |
|---|---|
| Faktor II | 0.3 bis 50 E/ml; |
| Faktor VII | 0.3 bis 30 E/ml; |
| Faktor IX | 0.5 bis 60 E/ml; |
| Faktor X | 0.4 bis 60 E/ml. |

Besonders bevorzugt sind die Bereiche 0.5 bis 25, 0.5 bis 15, 0.7 bis 30 und 0.6 bis 30 für die Faktoren II, VII, IX beziehungsweise X.

Als Konzentrat wird üblicherweise eine Lösung bezeichnet, die Wirkstoffe - also im vorliegenden Fall Gerinnungsfaktoren - in höherer Konzentration enthält als die natürlichen Ausgangsmaterialien.

**Tabelle I**

| Zusätze | | | | | | Gerinnungsaktivität nach Erhitzen (10 h, 60°C) in % bezogen auf das Ausgangsmaterial | | | |
|---|---|---|---|---|---|---|---|---|---|
| Saccharose g/100 g | Glycin mol/l | Ca-Ionen mmol/l | EDTA mmol/l | AT III E/ml | Heparin USP-E/ml | F II | F VII | F IX | F X |
| 60 | 2 | - | - | - | - | 83 | 58 | 25 | 43 |
| 60 | 2 | 6,25 | - | - | - | 47 | akt.* | akt.* | 40 |
| 60 | 2 | - | 5 | - | - | 73 | 61 | 41 | 50 |
| 60 | 2 | 25 | 5 | 0,2 | 2 | 72 | 83 | 83 | 87 |
| 60 | 2 | - | - | 0,2 | 2 | 72 | 55 | 48 | 33 |

*akt. = aktiviert

Für die Weiterreinigung kann die erhitzte Lösung gegebenenfalls zentrifugiert werden. Verunreinigungen können durch Ausfallung mit Ammoniumsulfat und 30 - 45 % Sättigung entfernt werden.

Der Überstand kann dann an 0,04 bis 1,0 g Calciumophosphat pro 100 ml Lösung adsorbiert, das beladene Adsorbens gewaschen, mit Citratpuffer eluiert und das Eluat dialysiert werden.

Für die Anwendung am Menschen kann das Produkt einer Sterilfiltration unterworfen werden.

Eine nach diesem Verfahren erhältliche, von aktiven Viren freie Faktor II, VII, IX und X-Präparation stellt im besonderen den Gegenstand der Erfindung dar.

Zur Erhöhung der Lagerstabilität ist es zweckmäßig, der Präparation Protein-stabilisierende Substanzen, beispielsweise Proteine, Aminosäuren oder Kohlenhydrate, zuzusetzen. Schließlich kann das dieser Behandlung unterzogene Präparat in gefriergetrockneter Form zur Verfügung gestellt werden, wobei der Zusatz von Antikoagulantien, wie beispielsweise Heparin, vorteilhaft sein kann.

Das erfindungsgemäße Produkt, speziell in Form eines Konzentrates, ist, in einer für die pharmazeutische Verabreichung geeigneten Lösung, ein Arzneimittel für die Behandlung von Koagulopathien und kann intravenös, vorteilhaft als Infusion, zur Therapie und Prophylaxe von durch Faktoren-Mangel bedingten Blutungen eingesetzt werden.

In Form eines lyophilisierten, gerinnungsaktiven Plasmas deckt das pasteurisierte Produkt die Indikationen von fresh-frozen-plasma ab, z. B. beim Auffüllen des Blutvolumens, Aufrechterhaltung des onkotischen Drucks, Blutverlust nach großen Operationen und Unfällen sowie die Behandlung der verschiedenen Formen des Schocks und die Erstversorgung von Polytraumatisierterten.

Die Erfindung soll an den nachstehenden Beispielen erläutert werden.

**Beispiel 1**

1000 ml Human-Citratplasma wurden unter Rühren auf 25 - 30°C erwärmt und mit 20 m. einer Lösung, die 1,86 g EDTA enthielt und mit 2 n NaOH auf pH 7,8 eingestellt worden war, versetzt. 10 min später wurden in die auf 30°C erwärmte Lösung 1000 g Saccharose langsam eingerührt. Nach Lösen der Saccharose wurden 150 g kristallines Glycin langsam und unter Rühren zugegeben; nachdem sich das vollständig gelöst hatte, wurden 25 ml 1 molarer $CaCl_2$-Lösung zugegeben und die Lösung mit 2 N NaOH auf pH 7,2 eingestellt. Die durch die Stabilisatoren auf 1,7 l vermehrte Lösung wurde im Wasserbad 10 Stunden auf 60°C erhitzt und war auch nach dem Erhitzen klar.

Entfernung der Stabilisatoren:

Nach dem Erkalten der pasteurisierten Lösung wurde dies über einen 3,0 und 1,2 µ Sartorius-Membran-Filter klärfiltriert; dann wurde die Lösung mit einem Citrat-NaCl-Puffer (0,01 mol/l Tri-NaCitrat pH 7,5; 0,06 mol/l NaCl) auf 5 l verdünnt und auf einem Ultrafilter unter gleichzeitigem Konzentrieren 3-mal gegen 5 l eines Puffers der gleichen Zusammensetzung dialysiert und auf 1000 ml konzentriert.

Nach Erreichen des Plasma-Ausgangsvolumens von 1000 ml wurde die klare Lösung über Sartorius-Membran-Filter klär- und sterilfiltriert, in 50 ml-Volumina abgefüllt und lyophilisiert. In dem mit 50 ml destilliertem Wasser rekonstituierten Trockengut wurden folgende Aktivitäten, bezogen auf ein Standard-Human-Plasma, gefunden; F II 87 %, F VII 81 %, F IX 83 %, F X 80 %.

**Beispiel 2**

1. Gewinnung der Prothrombin-Faktoren durch Adsorption an DEAE-Sephadex® und Elution

550 l Citratplasma wurden mit DEAE-Sephadex® A 50 (0,6 g/l) versetzt, das in physiologischer

Kochsalzlösung 5 mmol/l EDTA enthielt, und pH 6 äquilibriert worden war. Die Suspension wurde zur Adsorption 60 min bei 15°C gerührt. Nach Sedimentation des Adsorbens wurde der Überstand abgehebert und das DEAE-Sephadex® mit den adsorbierten Faktoren mit 50 l physiologischer Kochsalzlösung, die 5 mmol/l EDTA enthielt, solange gewaschen, bis es frei von verunreinigenden Proteinen war. Dann wurde das Adsorbens mit 7 l 1 mol/l NaCl, pH 8, enthaltend 5 mmol/l EDTA, gerührt, das Adsorbens durch Zentrifugation abgetrennt und verworfen.

2. Pasteurisierung

Zu dem Überstand wurden 0,2 E AT III/ml und 2 E/ml Heparin gegeben. Dann wurden 1 050 g festes Glycin zugesetzt und, nachdem sich dieses gelöst hatte, 25,7 g $CaCl_2 \cdot 2H_2O$ entsprechend einer Endkonzentration von 25 mmol/l. Nach Erreichen eines konstanten pH-Wertes von 7,2 wurde die Lösung auf 30 bis 37°C erhitzt und 10,5 kg Saccharose zugesetzt (Endkonzentration etwa 60 g/100 g Lösung). Nachdem der pH-Wert dieser hochviskosen Lösung noch einmal auf 7,2 eingestellt worden war, wurde die Lösung im Wasserbad 10 Stunden auf 60°C erhitzt.

3. Abtrennung unerwünschter Proteine durch Fällung mit Ammoniumsulfat

Die pasteurisierte Lösung, etwa 14 l, wurde auf Raumtemperatur abgekühlt und zu 31,5 l destilliertem Wasser gegeben, die 334 g $CaCl_2 \cdot 2H_2O$, entsprechend einer Endkonzentration von 50 mmol/l, enthielten.
In diese Lösung der Prothrombin-Faktoren wurden bei pH 7,5 24,5 l gesättigte Ammoniumsulfatlösung eingebracht, die Fällung abzentrifugiert und verworfen.

4. Hochreinigung der Prothrombinfaktoren durch Adsorption an Calciumphosphat

Der Überstand, die Prothrombinfaktoren enthaltend, wurde mit 700 g festem $Ca_3(PO_4)_2$ bei pH 7,6 versetzt und 20 bis 30 Minuten bei Raumtemperatur gerührt. Das Calciumphosphat wurde durch Zentrifugation gewonnen und dreimal mit 20 l einer Lösung von 0,5 mol/l NaCl und 1 g/100 ml Glycin, pH 7,6 gewaschen.
Die Elution der Prothrombinfaktoren vom Adsorbens erfolgte mit 750 ml eines Puffers, der 0,2 mol/l Trinatriumcitrat, 0,15 mol/l NaCl, 1 g/100 ml Glycin, 0,6 E/ml AT III und 28 E/ml Heparin enthielt (pH etwa 7,5), und Rühren (20 Minuten) bei Raumtemperatur. Das Eluat wurde durch Zentrifugation bei 3 000 g von groben Partikeln befreit.

5. Konfektionierung

Der Überstand wurde mit 0,15 g/100 ml Aerosil versetzt und durch Ultrazentrifugation bei 30 000 g von Feststoffen befreit. Zum klaren Überstand wurden etwa 0,6 g Humanalbumin auf 100 ml Flüssigkeit gegeben, auf pH 6,8 eingestellt und 3 Stunden gegen 50 l physiologische Kochsalzlösung dialysiert. Die Lösung auf etwa 60 E F IX, 60 E F II, 50 E F X und 25 E F VII eingestellt, sterilfiltriert, in 5 ml-Volumen abgefüllt und lyophilisiert.

**Patentansprüche**

1. Verfahren zur Herstellung einer praktisch virusfreien und hepatitissicheren Präparation der Blutgerinnungsfaktoren II, VII, IX und X durch Erwärmen einer diese Faktoren enthaltende wässrige Flüssigkeit, gegebenenfalls in Gegenwart einer Aminosäure und/oder eines Saccharids oder Zuckeralkohols, dadurch gekennzeichnet, daß in Gegenwart von Calciumionen und einem Chelat-Bildner und gegebenenfalls von Antithrombin III und/oder Heparin erwärmt wird.

2. Verfahren nach Anspruch 1, dadurch gekennzeichnet, daß die Konzentration von Calciumionen 1 - 50 mmol/l, vorzugsweise 25 - 50 mmol/l, ist.

3. Verfahren nach Anspruch 1, dadurch gekennzeichnet, daß der Chelat-Bildner eine aliphatische Aza-tri- oder -tetra-Carbonsäure mit 6 bis 20 Kohlenstoffatomen und 1 oder 2 Stickstoffatomen oder ihr lösliches Alkalisalz ist.

4. Verfahren nach Anspruch 1, dadurch gekennzeichnet, daß die Konzentration des Chelat-Bildners 1 bis 20, vorzugsweise 5 mmol/l ist.

5. Verfahren nach Anspruch 1, dadurch gekennzeichnet, daß in Gegenwart von 0,2 - 2 E/ml Antithrombin III, 2 - 20 USP-E/ml Heparin, 25 - 50 mmol/l Calciumionen, 1 - 20 mmol/l EDTA, 1 - 3 mol/l mindestens einer der Aminosäuren Glycin, alpha- oder beta-Alanin, Lysin, Leucin, Valin, Asparagin, Serin, Hydroxyprolin, Prolin, Glutamin, alpha-, beta- oder gamma-Aminobuttersäure, vorzugsweise Glycin, und 20 bis 60 g/100 g Lösung eines Mono- oder Oligosaccharids oder Zuckeralkohols vorzugweise 1 bis 3 mol/l Glycin und 20 bis 60 g/100 g Lösung Saccharose, auf eine Temperatur zwischen 30°C und 100°C, vorzugsweise 60°C bis 100°C, 1 Minute bis 48 Stunden, vorzugsweise 8 - 12 Stunden, erwärmt wird.

6. Verfahren nach Anspruch 1, dadurch gekennzeichnet, daß die wässrige Flüssigkeit Citratplasma oder eine Plasma- oder Placenta-Fraktion ist.

7. Verfahren nach Anspruch 1, dadurch gekennzeichnet, daß die wässrige Flüssigkeit ein Konzentrat der Faktoren II, VII, IX und X ist.

**Claims**

1. A process for the preparation of a virtually virus-free and hepatitis-safe product of blood coagulation factors II, VII, IX and X by heating an aqueous liquid containing these factors, if appropriate in the presence of an aminoacid and/or a saccharide or sugar-alcohol, which comprises heating the solution in the presence of calcium ions and a chelating agent and, if appropriate, antithrombin III and/or heparin.

2. The process as claimed in claim 1, wherein the concentration of calcium ions is 1 - 50 mmol/l, preferably 25 - 50 mmol/l.

3. The process as claimed in claim 1, wherein the chelating agent is an aliphatic aza-tri- or -tetra-carboxylic acid with 6 to 20 carbon atoms and 1 or 2 nitrogen atoms, or a soluble alkali metal salt thereof.

4. The process as claimed in claim 1, wherein the concentration of the chelating agent is 1 to 20, preferably 5, mmol/l.

5. The process as claimed in claim 1, wherein the solution is heated in the presence of 0.2 - 2 units/ml of antithrombin III, 2 - 20 USP units/ml of heparin, 25 - 50 mmol/l of calcium ions, 1 - 20 mmol/l of EDTA 1-3 mol/l of at least one of the aminoacids glycine, alpha- or beta-alanine, lysine, leucine, valine, asparagine, serine, hydroxyproline, proline, glutamine or alpha-, beta- or gamma-aminobutyric acid, preferably glycine, and 20 to 60 g/100 g of a solution of a mono- or oligo-saccharide or sugar-alcohol, preferably 1 to 3 mol/l of glycine and 20 to 60 g/100 g of solution of sucrose, at a temperature between 30°C and 100°C, preferably at 60°C to 100°C, for 1 minute to 48 hours, preferably 8 - 12 hours.

6. The process as claimed in claim 1, wherein the aqueous liquid is citrated plasma or a plasma or placenta fraction.

7. The process as claimed in claim 1, wherein the aqueous liquid is a concentrate of factors II, VII, IX and X.

**Revendications**

1. Procédé de production d'une préparation des facteurs de coagulation du sang II, VII, IX et X pratiquement exempte de virus et ne présentant pas de danger d'hépatite, par chauffage d'un liquide aqueux contenant ces facteurs, éventuellement en présence d'un aminoacide et/ou d'un saccharide ou alcool de sucre, caractérisé en ce que l'on chauffe en presence d'ions calcium et d'un chélatant et éventuellment d'antithrombine III et/ou d'héparine.

2. Procédé selon la revendication 1, caractérisé en ce que la concentration d'ions calcium est de 1 à 50 mmoles/l, de préférence de 25 à 50 mmoles/l.

3. Procédé selon la revendication 1, caractérisé en ce que le chélatant est un aza-tri- ou -tétra-acide carboxylique ayant de 6 à 20 atomes de carbone et 1 ou 2 atomes d'azote ou son sel de métal alcalin soluble.

4. Procédé selon la revendication 1, caractérisé en ce que la concentration du chélatant est de 1 à 20, de préférence de 5 mmoles/l.

5. Procédé selon la revendication 1, caractérisé en ce que l'on chauffe en présence de 0,2 à 2 U/ml d'antithrombine III, 2 à 20 U USP/ml d'héparine, 25 à 50 mmoles/l d'ions calcium, 1 à 20 mmoles/l d'EDTA, 1 à 3 moles/l d'au moins un des aminoacides glycine, alpha- ou béta-alanine, lysine, leucine, valine, asparagine, sérine, hydroxyproline, proline, glutamine, acide alpha-, béta- ou gamma-aminobutyrique, de préférence glycine, et 20 à 60 g de d'un mono- ou oligosaccharide ou alcool de sucre pour 100 g de solution, de préférence 1 à 3 moles/l de glycine et 20 à 60 g de saccharose pour 100 g de solution, à une température comprise entre 30°C et 100°C, de préférence 60°C et 100°C, pendant 1 minute à 48 heures, de préférence 8 à 12 heures.

6. Procédé selon la revendication 1, caractérisé en ce que le liquide aqueux est du plasma citraté ou une fraction de plasma ou de placenta.

7. Procédé selon la revendication 1, caractérisé en ce que le liquide aqueux est un concentré de facteurs II, VII, IX et X.